# EUROPEAN PATENT SPECIFICATION

(11) **EP 3 937 992 B1**
(45) Date of publication and mention of the grant of the patent: **07.10.2026**
(21) Application number: 20717981.3
(22) Date of filing: 11.03.2020
(51) Int. Cl.: A61L 2/18, B65G 53/46, B65G 51/34, B65G 53/24

(54) **APPARATUS TO FEED LOOSE OBJECTS TO A TREATMENT MACHINE AND CORRESPONDING FEED METHOD**
VORRICHTUNG ZUM ZUFÜHREN LOSER GEGENSTÄNDE ZU EINER BEHANDLUNGSMASCHINE UND ENTSPRECHENDES ZUFÜHRVERFAHREN
APPAREIL POUR ALIMENTER DES OBJETS EN VRAC DANS UNE MACHINE DE TRAITEMENT ET PROCÉDÉ D'ALIMENTATION CORRESPONDANT

(30) Priority: 14.03.2019 IT 201900003773
(43) Date of publication of application: 19.01.2022
(73) Proprietor: Icos Pharma S.p.A., 33080 Zoppola (IT)
(72) Inventor: BENI, Stefano, 33170 PORDENONE (IT)
(74) Representative: Petraz, Gilberto Luigi
(86) International application number: PCT/IT2020/050059
(87) International publication number: WO 2020/183510

(56) References cited:
- EP-A1- 3 064 226
- WO-A2-2018/225105
- US-A1- 2002 187 011
- US-B2- 8 784 013

## Description

### FIELD OF THE INVENTION

The present invention concerns an apparatus to feed loose objects to a treatment machine and the corresponding feed method. In particular, the treatment machine can be used in the hospital, pharmaceutical, or foodstuffs sector, or in the industrial sector in the production of said loose objects.

By treatment here we generally mean at least one washing and/or sterilization operation, with possible final drying.

The loose objects can be, for example, caps for closing test tubes for laboratory analysis, caps for closing vials of drugs, or small instruments or objects used in operating rooms or laboratories, or other.

### BACKGROUND OF THE INVENTION

It is known that in the hospital, or pharmaceutical, field, or in other sectors, such as the foodstuff sector, it is necessary to treat loose objects, even with small sizes, such as, for example, caps for closing test tubes intended to contain substances to be subjected to laboratory analysis, or small instruments or objects used in operating rooms or laboratories.

It is known to treat these loose objects in treatment machines provided with a sealed chamber in which a rotating drum receives the objects to be treated from a so-called "dirty" loading side, carries out their treatment, and unloads them from a so-called "sterile" unloading side, where they are generally packaged into sterile packages.

Some examples of machines to treat loose objects are described for example in documents WO-A-2015150581, PCT/IT2018/050098, WO-A-2018/225105 and EP-A-3064226 in the name of the Applicant.

These known treatment machines are provided with rotating drums partitioned into containing sectors, each containing a desired quantity of loose objects to be poured into the individual packages after the treatment.

As is known, see for example documents WO-A-2015150581 and PCT/IT2018/050098, these treatment machines comprise apparatuses to feed loose objects which load the loose objects into the treatment chamber provided with a drum by means of a loading zone located above the treatment chamber. A containing sector of the rotating drum faces, on each occasion, toward the loading zone, so as to introduce a correct quantity of loose objects into the containing sector.

The feed of the loose objects through the loading zone can occur manually, or by means of a pneumatic system that sucks in the objects from a basket and sends them to a loading container, for example a metering cylinder, located in an upper zone of the treatment machine, in correspondence with the loading zone of the treatment chamber provided above the treatment machine.

Some examples of pneumatic systems to transport objects, which however prove to be unsuitable for use in machines to treat objects, are described for example in documents US-B-8784013 and US-A-2002/187011.

The feed operations as above of the treatment machines with rotating drum could be similar also for treatment machines with a treatment chamber in which there is no rotating drum, but in which, for example, the loose objects to be treated are housed in other containing elements.

Over the years, the need has arisen to obtain treatment machines with increasingly compact, optimized, safe, reliable and controllable apparatuses to feed loose objects, particularly in the field of application of the pharmaceutical industry.

There is therefore the need to perfect an apparatus to feed loose objects to a treatment machine that can overcome and improve the known feed apparatuses as above.

In particular, one purpose of the present invention is to provide an apparatus to feed loose objects to a treatment machine which is effective, optimized and automated.

It is also a purpose of the present invention to provide an apparatus to feed loose objects to a treatment machine which has a small bulk and an optimized energy use.

It is also a purpose to define a method to feed loose objects to a treatment machine which is efficient and optimized, and which increases the productivity of the treatment machine by reducing downtimes between one loading of loose objects and the next.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, the present invention concerns a feed apparatus to feed loose objects to a treatment machine able to treat loose objects and comprising a treatment chamber provided with a loading zone of the loose objects.

Furthermore, according to the invention, the feed apparatus comprises:
- a source of loose objects;
- a metering device which has a loading side connected to the source of loose objects by means of a feed pipe of the loose objects and an unloading side on which an interception valve is mounted, wherein the metering device is configured to accommodate a determinate quantity of loose objects;
- an introduction assembly connected to the metering device by means of a sending pipe connected to an outlet member present on the unloading side of the metering device, wherein the introduction assembly is configured to introduce the loose objects, into the loading zone, directly in the treatment chamber;
- a pneumatic feed circuit selectively drivable alternately in a removal condition and in a condition for sending the loose objects respectively from the source to the metering device, through the feed pipe, and from the metering device to the introduction assembly;
- a pneumatic device connected to the pneumatic feed circuit and configured to suck in loose objects from the source to the metering device in the removal condition and to blow loose objects from the metering device to the introduction assembly.

The pneumatic feed circuit comprises:
a suction sub-circuit which includes the feed pipe, a first suction pipe connected to the loading side of the metering device, a second suction pipe connected to an external environment and a main suction pipe connected on one side to the first suction pipe and to the second suction pipe and on another side connected to a suction door of the pneumatic device, wherein respective valves are mounted on the first suction pipe and on the second suction pipe, wherein the suction sub-circuit is provided with a first suction intake, provided in the source, associated with the pneumatic device and connected to the feed pipe, and with a second suction intake, provided terminally on the second suction pipe, associated with the pneumatic device and connected to the external environment, wherein the valves are configured to alternately close the second suction pipe and the first suction pipe respectively in the removal condition and in the condition for sending the loose objects.

Advantageously, the apparatus to feed loose objects to a treatment machine provides an optimized feed of the loose objects as above, reducing costs and bulk compared to known apparatuses. In fact, the pneumatic feed circuit as above is managed by a single pneumatic device in an automated manner and substantially eliminating the manual intervention of a user.

Furthermore, the apparatus to feed loose objects in accordance with the present invention is considerably optimized in energy terms, actively exploiting both the suction step and also the blowing step of the pneumatic device and, consequently, the apparatus can have a more compact and contained overall bulk, reducing production costs and the spaces necessary for its use.

The present invention also concerns a treatment plant that overcomes the limitations of the state of the art and eliminates the defects present therein, comprising a treatment machine able to treat loose objects and the apparatus to feed loose objects as above to feed the loose objects to be treated to the treatment machine.

According to one embodiment, in the treatment plant the source of loose objects and the metering device are installed in a common feed area substantially opposite the treatment machine. In this way, the treatment plant has a compact and contained bulk.

The present invention also concerns a feed method to feed loose objects to a treatment machine able to treat loose objects in a treatment chamber provided with a loading zone of the loose objects.

The method as above comprises:
- suction of a metered quantity of loose objects from a source of loose objects in a metering device by means of a pneumatic feed circuit driven in a condition of removing the loose objects from the source to the metering device using a pneumatic device connected to the pneumatic feed circuit to suck in loose objects from the source to the metering device;
- blowing the metered quantity of loose objects present in the metering device from the metering device by means of the pneumatic feed circuit driven in a condition for sending the loose objects from the metering device to an introduction assembly which introduces the loose objects, in the loading zone, directly into the treatment chamber, using the pneumatic device to blow the loose objects from the metering device to the introduction assembly.

In the present method, the pneumatic feed circuit comprises: a suction sub-circuit which includes the feed pipe, a first suction pipe connected to the loading side of the metering device, a second suction pipe connected to an external environment and a main suction pipe connected on one side to the first suction pipe and to the second suction pipe and on another side connected to a suction door of the pneumatic device, wherein respective valves are mounted on the first suction pipe and on the second suction pipe, wherein the suction sub-circuit is provided with a first suction intake, provided in the source, associated with the pneumatic device and connected to the feed pipe and with a second suction intake, provided terminally on the second suction pipe, associated with the pneumatic device and connected to the external environment, wherein the valves are configured to alternately close the second suction pipe and the first suction pipe respectively in the removal condition and in the condition for sending the loose objects.

Advantageously, the method to feed loose objects is efficient and optimized in terms of economy and energy.

In particular, this method, since it is substantially completely automated and managed in a single pneumatic feed circuit, allows to reduce the downtimes between one load of loose objects and the subsequent one and, therefore, between a removal condition and a subsequent condition for sending the metered loose objects.

### BRIEF DESCRIPTION OF THE DRAWINGS

These and other aspects, characteristics and advantages of the present invention will become apparent from the following description of some embodiments, given as a non-restrictive example with reference to the attached drawings wherein:
- fig. 1 is an example diagram of an apparatus to feed loose objects to a treatment machine in accordance with the present invention;
- fig. 2 is a perspective view of an apparatus to feed loose objects to a treatment machine in accordance with the present invention;
- fig. 3 is a partly sectioned view of a detail of the feed apparatus of fig. 2;
- fig. 4 is a perspective view of a detail of the feed apparatus of fig. 2;
- fig. 5 is a sectioned perspective view of a detail of the feed apparatus of fig. 2.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the various embodiments of the invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing the embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

With reference to the attached drawings, these show a feed apparatus 10 to feed loose objects 11 to a treatment machine 14 able to treat the loose objects 11. Here and in the present description, with the terms "treatment" and "treat" we mean one or more washing and/or sterilization operations, with possible final drying. As part of the washing, it is possible to carry out a pre-wash, a wash with water or a wash with chemical agents, a combination of these washes, a rinse with hot or cold water. As part of the treatment, a possible thermal disinfection operation is also contemplated. As part of the treatment, it is also possible to surface treat the loose objects 11, for example by means of silicone coating; the silicone coating can be carried out for example in the context of the washing operation. A possible example is a treatment which includes washing and drying. Another possible example of treatment provides washing, sterilization and drying. In these examples, the surface treatment of the loose objects 11, for example silicone coating, can be carried out at the same time as or after washing, before sterilization when provided. Yet another example of a treatment provides sterilization and drying.

The treatment machine 14 comprises a treatment chamber 12 provided with a loading zone 13 for the loose objects 11.

In particular, the loose objects 11 to be treated are able to be housed in the treatment chamber 12.

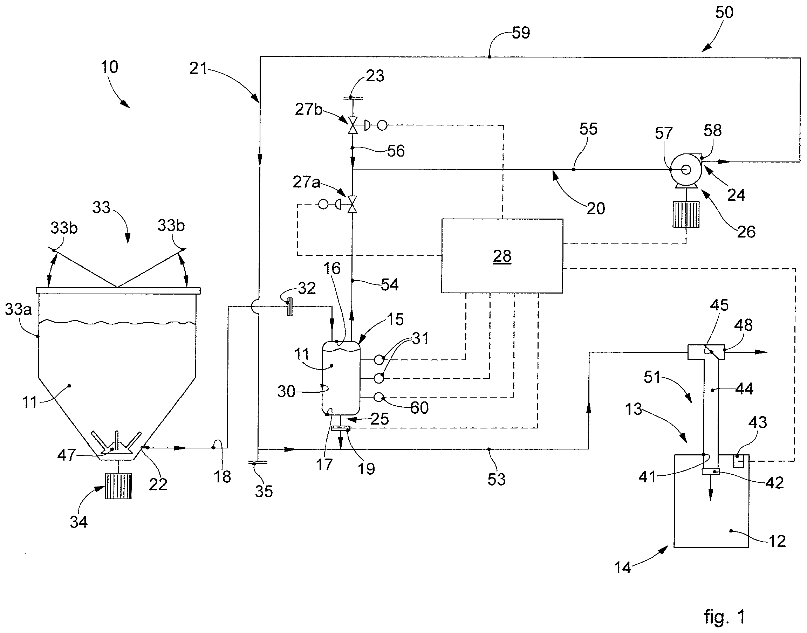

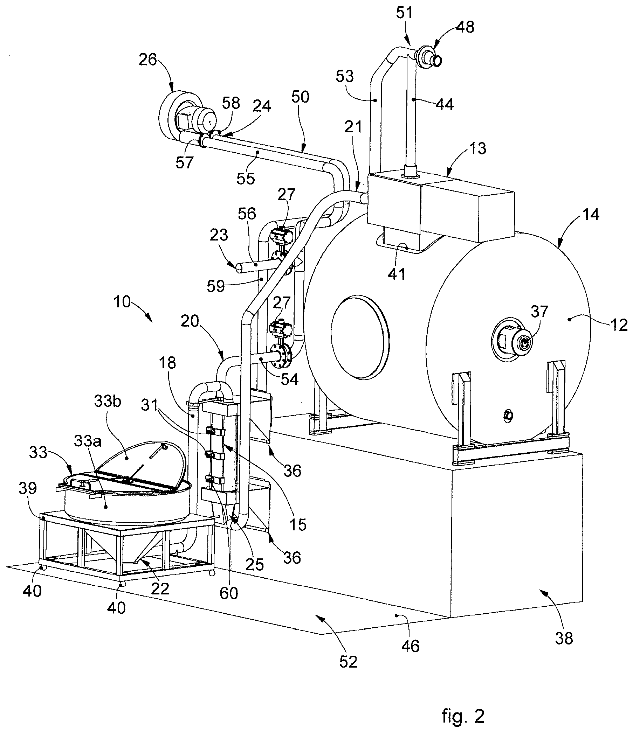

According to one aspect of the invention, with reference to figs. 1-2, the feed apparatus 10 also comprises a source 33 of loose objects 11 suitable to contain the loose objects 11 to be treated.

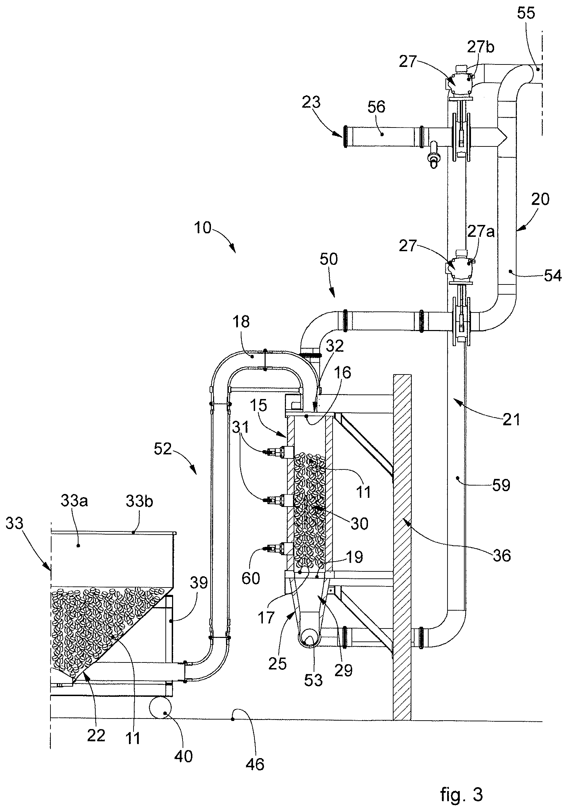

According to one aspect of the invention, with reference for example to figs. 3-4, the feed apparatus 10 also comprises a metering device 15 which has a loading side 16 connected to the source 33 of loose objects 11 by means of a feed pipe 18 of the loose objects 11 and a unloading side 17 to which an interception valve 19 is associated.

In particular, the metering device 15 is configured to accommodate a determinate quantity of loose objects 11.

According to one embodiment, the unloading side 17 can be selectively opened by means of the interception valve 19.

According to another aspect of the invention, the feed apparatus 10 also comprises an introduction assembly 51 connected to the metering device 15 by means of a sending pipe 53 connected to an outlet member 25 present on the unloading side 17 of the metering device 15.

The introduction assembly 51 is configured to introduce the loose objects 11, in the loading zone 13, directly into the treatment chamber 12.

According to another aspect of the invention, the feed apparatus 10 also comprises a pneumatic feed circuit 50 selectively drivable alternately in a removal condition and in a condition for sending the loose objects 11 respectively from the source 33 of loose objects 11 to the metering device 15, through the feed pipe 18, and from the metering device 15 to the introduction assembly 51.

According to another aspect of the invention, the feed apparatus 10 also comprises a pneumatic device 26 connected to the pneumatic feed circuit 50 and configured to suck in loose objects 11 from the source 33 of loose objects 11 to the metering device 15 in the removal condition and to blow loose objects 11 from the metering device 15 to the introduction assembly 51.

The pneumatic device 26 can be, for example, a blower, a compressor or other pumping device provided with a suction side or suction door 57 and a blowing side or blowing door 58.

The pneumatic feed circuit 50 comprises a suction sub-circuit 20 which includes:
- the feed pipe 18,
- a first suction pipe 54 connected to the loading side 16 of the metering device 15,
- a second suction pipe 56 connected to an external environment,
- a main suction pipe 55 connected on one side to the first suction pipe 54 and to the second suction pipe 56, and on another side connected to the suction door 57 of the pneumatic device 26.

The suction sub-circuit 20 also comprises respective valves 27 mounted on the first suction pipe 54 and on the second suction pipe 56.

The valves 27 can be, for example, solenoid valves.

The suction sub-circuit 20 is also provided with:
- a first suction intake 22, provided in the source 33 of loose objects 11, associated with the pneumatic device 26 and connected to the feed pipe 18;
- a second suction intake 23, provided terminally on the second suction pipe 56, associated with the pneumatic device 26 and connected to the external environment.

The external environment can advantageously be a sterile chamber, or a chamber with suitably treated air to manage and transport the loose objects 11 to be treated. In this way, the loose objects are transported and fed to the loading zone 13 with air compatible and suitable for the treatment environment provided in the treatment chamber 12.

According to one embodiment, the valves 27 are configured to alternately close the second suction pipe 56 and the first suction pipe 54 respectively in the removal condition and in the condition for sending the loose objects 11.

In particular, the first suction intake 22 can be configured to generate a depression inside the metering device 15 so as to suck in the loose objects 11 from the source 33 of loose objects 11, through the feed pipe 18, and to fill the metering device 15 up to the desired level.

Furthermore, the second suction intake 23 can be configured to suck in from the external environment when the metering device 15 has reached the desired level, thus ensuring continuous functioning of the pneumatic device 26.

According to another embodiment, the pneumatic feed circuit 50 comprises a blowing sub-circuit 21 which includes:
- the sending pipe 53,
- a delivery intake 24 present on a blowing door 58 of the pneumatic device 26,
- a blowing pipe 59 connected to the sending pipe 53 and to the outlet member 25 present on the unloading side 17 of the metering device 15.

According to one embodiment, the sending pipe 53 ends with an outlet portion 48 configured to vent the blowing of the pneumatic device 26. The outlet portion 48, for example, vents the air blown by the pneumatic device 26.

The outlet portion 48 is directly connected with the blowing door 58 of the pneumatic device 26 through the blowing pipe 59 and the sending pipe 53.

The outlet portion 48 can be disposed above the loading zone 13.

The outlet portion 48 can be disposed parallel to the loading zone 13.

According to another embodiment, the blowing sub-circuit 21 also includes the interception valve 19 mounted on the outlet member 25, the interception valve 19 being configured to close the outlet member 25 in the removal condition and to open the outlet member 25 in the sending condition.

According to one embodiment, the interception valve 19 is selectively openable/closable in synchrony with the respective opening/closing of the valve 27b mounted on the second suction pipe 56 and closing/opening of the valve 27a mounted on the first suction pipe 54.

Advantageously, the feed apparatus 10 uses both the suction side, that is, the suction door 57, and also the blowing side, that is, the blowing door 58, of the pneumatic device 26 respectively by means of the suction sub-circuit 20 and the blowing sub-circuit 21 optimizing the feed apparatus 10 in terms of energy and economy.

Consequently, with a single pneumatic feed circuit 50 it is possible both to remove loose objects 11 from the source 33 and also send them, once correctly metered, to the treatment machine 14 reducing to a minimum the manual intervention of a user and, therefore, the possible contaminations of the external environment.

Furthermore, the use of the pneumatic feed circuit 50 allows to optimize the possibilities of use of the pneumatic device 26, maximizing the energy use thereof. According to one embodiment, the outlet member 25 can provide a channeling hopper 29 disposed below the unloading side 17 and configured to channel the loose objects 11, in the sending condition, into the sending pipe 53.

According to one embodiment, the channeling hopper 29 can have a truncated cone or truncated pyramid shape converging toward the sending pipe 53. Advantageously, this shape, together with the depression created as a result of the air blown by the pneumatic device 26 inside the blowing sub-circuit 21 and the opening of the interception valve 19 allow a complete, uniform and efficient discharge of the loose objects 11 from the metering device 15 and their consequent feed to the treatment chamber 12.

According to one embodiment, the feed apparatus 10 comprises a command and control unit 28 configured to control the pneumatic feed circuit 50 in correlation with the pneumatic device 26 to selectively determine at least the removal condition and the sending condition.

According to one embodiment, the command and control unit 28 is configured to control the opening/closing synchrony of the interception valve 19 with respect to the valve 27b mounted on the second suction pipe 56 and with respect to the valve 27a mounted on the first suction pipe 54.

The command and control unit 28 can also command the selective ignition of the pneumatic device 26.

In this way, the command and control unit 28 allows to manage in an automatic and automated manner the loose objects 11 to be fed to the treatment machine 14, making the feed apparatus 10 more efficient and automated.

According to one embodiment, the blowing sub-circuit 21, the suction sub-circuit 20 can be made at least partly of transparent material so as to visually control the correct sending and removal of the loose objects 11 respectively in the removal condition and in the condition for sending loose objects 11.

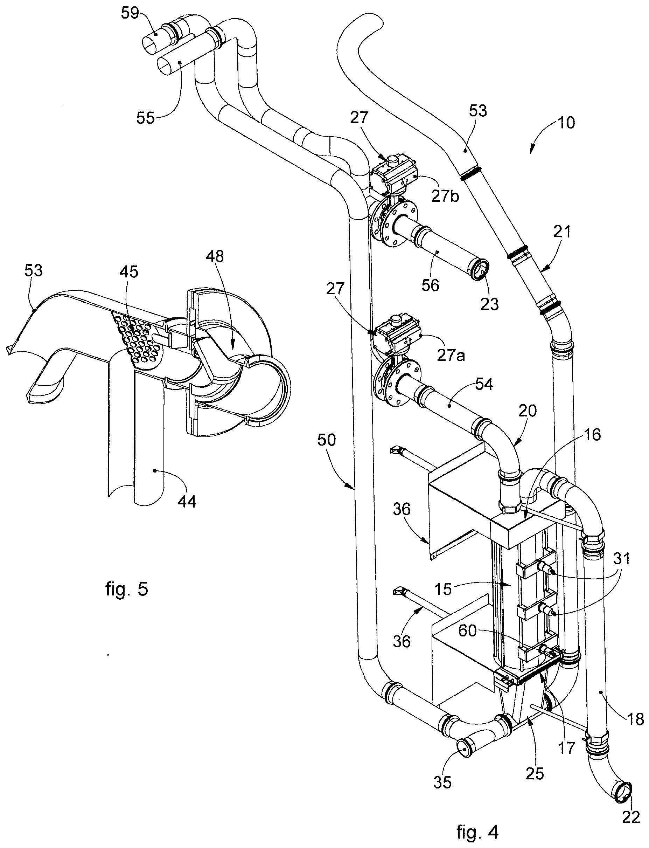

According to one embodiment, the blowing sub-circuit 21 can comprise, for example, an inspection intake 35 (figs. 1 and 4) in the sending pipe 53 made at least partly of transparent material and disposed in correspondence with the outlet member 25. The inspection intake 35, for example, allows to check whether any obstruction or any residual loose objects 11 are present underneath the unloading side 17 of the metering device 15, in the blowing sub-circuit 21. The inspection intake 35 also allows to facilitate and accelerate the maintenance operations of the feed apparatus 10.

According to one embodiment, the source 33 of loose objects 11 and the metering device 15 are installed in a common feed area 52 substantially opposite the treatment machine 14. In this way, the feed apparatus 10 is more compact and easier to maintain since the metering device 15 and the source 33 are installed in the feed area 52 in a zone that is easy to access and check for an operator, since it is disposed opposite the treatment machine 14. Consequently, the disposition of the source 33 and the metering device 15 facilitates access to them by an operator and therefore simplifies routine maintenance operations, such as cleaning and inspection of the feed apparatus 10, and extraordinary maintenance operations, such as the replacement of components of the apparatus 10. Advantageously, for inspection purposes, the metering device 15 can be transparent or have at least a portion or window that is transparent, or in any case suitable for an optical/visual inspection of the interior.

Advantageously, the feed area 52 coincides with the external environment from which the second suction intake 23 draws the air to send the loose objects 11 to the treatment chamber 12 in the sending condition. In this way, the air processed by the pneumatic device 26 is always drawn, both in the case of the first suction intake 22 and also in the case of the second suction intake 23, from a controlled environment, for example sterile, thus preventing infiltrations of harmful substances that could invalidate the correct treatment inside the treatment chamber 12.

According to some embodiments, the metering device 15 can comprise a metering chamber 30.

The metering chamber 30 can be for example cylindrical.

The metering chamber 30 receives a metered quantity of loose objects 11 from the source 33 of loose objects 11 along the feed pipe 18, according to the loading needs of the treatment chamber 12.

In particular, the metered quantity of loose objects 11 can correspond to the quantity of loose objects 11 which at the end of the treatment process will be packed or packaged into sterile packages to be sent, for example, to laboratories or specialized centers that use them.

Depending on the size of the loose objects 11, there can be provided different filling levels of the metering chamber 30 of the metering device 15.

According to one embodiment, the metering device 15 can comprise one or more level sensors 31 along its longitudinal development. The level sensors 31, based on the filling achieved and on the type of loose objects 11 present in the metering device 15, send a signal to the command and control unit 28 to interrupt the removal of the loose objects 11 from the source 33 and, then, to proceed to the condition for sending them to the treatment chamber 12.

For example, the level sensors 31 can be optical or gravimetric sensors, or a combination of these two types.

According to one embodiment, the metering device 15 can comprise at least one sensor 60 which detects whether the metering chamber 30 is empty.

The sensor 60 can be associated with the bottom of the metering chamber 30 or with the unloading side 17.

For example, the sensor 60 can be optical or capacitive or gravimetric, or a combination thereof. The sensor 60 can be a presence, level or weight sensor or a combination thereof.

According to another embodiment, the feed pipe 18 can be provided with a blocking valve 32 which selectively obstructs it to prevent further removal of loose objects 11 to the metering device 15 when it reaches the correct filling level, that is, when passing from the removal to the sending condition.

According to a possible embodiment, the blocking valve 32 can be a guillotine valve provided terminally to the feed pipe 18 and associated with the loading side 16 of the metering device 15.

The blocking valve 32 can be connected to the command and control unit 28 which determines its selective drive.

According to one embodiment, the interception valve 19 associated with the unloading side 17 selectively opens the bottom of the metering device 15 when the metering chamber 30 is correctly filled with loose objects 11.

The interception valve 19 can be a guillotine or shutter valve. This solution allows the metered loading of the loose objects 11 into the treatment chamber 12.

The metering device 15 can be kept in a vertical position by means of a support frame 36.

According to some embodiments, the metering device 15 can be interposed between the treatment chamber 12 and the source 33 of loose objects 11.

According to one embodiment, the source 33 of loose objects 11 can be a container 33a for collecting the loose objects 11 configured to contain the loose objects 11 to be treated.

According to some embodiments, the metering device 15 is connected to the collection container 33a by means of the feed pipe 18.

According to a possible embodiment, the feed pipe 18 can connect the lower side of the collection container 33a, where the first suction intake 22 is provided, to the loading side 16 of the metering device 15.

The collection container 33a can have a truncated cone shape, so as to facilitate the transit of the loose objects 11 downward and therefore toward the feed pipe 18.

The collection container 33a can be provided with a lid 33b, for example with openable panels.

According to one embodiment, the collection container 33a (figs. 1 - 3) is equipped with shaker means 47 preferably made to rotate about an axis, for example the central vertical axis of the collection container 33a, by means of suitable drive means 34, so as to keep the loose objects 11 continuously in motion and allow their correct movement along the feed pipe 18.

The collection container 33a, whether it is fixed or rotatable, is supported by a suitable support structure 39.

According to one embodiment, the collection container 33a can be mobile in the feed area 52 by means of rolling elements 40, such as casters or suchlike.

The support structure 39 of the collection container 33a rests on and is supported by the rolling elements 40.

Furthermore, if the collection container 33a is mobile, the connection between the collection container 33a and the feed pipe 18 can be made by means of flexible and/or telescopic tubes and possibly by means of a rotatable joint to also allow the rotation of the collection container 33a on a support surface 46 in the feed area 52.

According to one embodiment, the loading zone 13 is provided at the top of the treatment chamber 12 and configured to feed the loose objects 11, which fall due to gravity, inside the treatment chamber 12.

According to one embodiment, the loading zone 13 can comprise, or consist of, a completely sealed introduction pre-chamber, for example formed by a sealed frame or box-like body. The pre-chamber is sealed in order to prevent non-sterile or, in general, not properly controlled, air from entering inside the treatment chamber 12, which could damage the treatment of the loose objects 11 inside the treatment chamber 12.

According to another embodiment, the loading zone 13 comprises a loading aperture 41 which allows to load the loose objects 11 inside the treatment chamber 12, and the sealed pre-chamber is disposed in correspondence with the aperture 41 in order to guarantee a sealed and controlled introduction environment.

The aperture 41 can be selectively closed or opened by means of a guillotine or shutter closing device disposed between the treatment chamber 12 and the pre-chamber.

According to one embodiment, the treatment machine 14 includes an unloading zone (not shown) of the loose objects 11 after the treatment, for example positioned on the opposite side of the treatment machine 14 with respect to the loading zone 13.

In particular, the unloading zone can be a sterile chamber, or a "clean room" with a controlled atmosphere.

The treatment chamber 12 comprises a support frame 38, by means of which, preferably, it is located at a certain height with respect to the support surface 46.

The treatment chamber 12 can contain, for example, a rotating drum in which the loose objects 11 are in turn contained during the treatment. In particular, the rotating drum can rotate around a shaft 37.

The drum can comprise a plurality of containing sectors disposed radially and intended to each contain the metered quantity of loose objects 11 to be treated.

Examples of such treatment chambers 12 with the drums as above are described in documents WO-A-2015150581, PCT/IT2018/050098 and EP-A-3064226 in the name of the Applicant.

According to one embodiment, the rotation of the drum can be commanded by the command and control unit 28 in cooperation with the command and control of the pneumatic feed circuit 50 and of the pneumatic device 26 at least in the removal condition and in the condition for sending the loose objects 11. In this way, for example, once a first containing sector has been filled, it is possible to proceed to the next one by rotating the drum.

The present invention can also be applied to different types of treatment chambers 12, as long as they require a preventive metering of the loose objects 11.

According to one embodiment, the present treatment machine 14 can also comprise a control device 43 (fig. 1) to verify the correct loading of the loose objects 11 metered into the treatment chamber 12.

The control device 43 can be located in correspondence with the loading aperture 41, so as to verify the correct loading of the loose objects 11, in the treatment chamber 12, for example in a containing sector positioned in correspondence with the loading zone 13.

The control device 43 can be for example a digital camera, an optical sensor or suchlike.

The control device 43 can be associated with the command and control unit 28 of the treatment machine 14.

According to one embodiment, the introduction assembly 51 comprises a connection pipe 44 transverse to the treatment chamber 12 and communicating with the inside of the treatment chamber 12 through the aperture 41.

The connection pipe 44 connects the sending pipe 53 to the treatment chamber 12 in the loading zone 13.

In particular, the connection pipe 44 is disposed in a T-shape with the sending pipe 53.

The connection pipe 44 can be provided with a stop valve 42, for example a guillotine valve, which can be selectively activated from an open position to a closed position, or vice versa.

Advantageously, the connection pipe 44 is telescopic, drivable to be selectively lengthened and retracted with respect to the treatment chamber 12 in order to be positioned, with one end portion thereof, inside the treatment chamber 12 itself, so as to introduce directly inside it the loose objects 11 to be treated. In this way, thanks to the feed directly inside the treatment chamber 12, the risk of unwanted spills or dispersions of loose objects 11 outside the treatment chamber 12 is prevented, with the guarantee, therefore, that all the loose objects 11 which are sent by the metering device 15 to the introduction assembly 51 are effectively introduced into the treatment chamber 12.

Furthermore, the selectively retractable and extendable telescopic connection pipe 44 allows, in its retracted position, the control device 43 to correctly verify without impediments the conditions of the treatment chamber 12 in the steps preceding and following the loading of the loose objects 11 inside it.

For example, the control device 43, when the connection pipe 44 is retracted, can check whether the containing sector to be loaded is correctly positioned and does not have loose objects 11 inside it.

According to one embodiment, as shown in fig. 5, the introduction assembly 51 comprises a deflector element 45 disposed before an outlet portion 48 of the sending pipe 53 configured to vent the blowing of the pneumatic device 26. The deflector element 45 deflects the loose objects 11 in the sending condition, toward the loading zone 13 before reaching the outlet portion 48.

According to one embodiment, the deflector element 45 can be a perforated plate that allows the passage of air toward the outlet portion 48 preventing the loose objects 11 from passing.

The deflector element 45 can advantageously produce a connection between the sending pipe 53 and the connection pipe 44 allowing the air to escape through the outlet portion 48 and the loose objects 11 to fall due to gravity along the connection pipe 44 into the treatment chamber 12.

According to one embodiment, the method to feed the loose objects 11 into the treatment machine 14 comprises:
- suction of a metered quantity of loose objects 11 from the source 33 of loose objects 11 in the metering device 15 by means of the pneumatic feed circuit 50 driven in the condition of removing the loose objects 11 from the source 33 to the metering device 15 using the pneumatic device 26 connected to the pneumatic feed circuit 50 to suck in the loose objects 11 from the source 33 to the metering device 15;
- blowing the metered quantity of loose objects 11, present in the metering device 15, from the metering device 15 by means of the pneumatic feed circuit 50 driven in the condition for sending the loose objects 11 from the metering device 15 to the introduction assembly 51 which introduces the objects loose 11, in the loading zone 13, directly into the treatment chamber 12, using the pneumatic device 26 to blow the loose objects 11 from the metering device 15 to the introduction assembly 51.

Substantially, therefore, once the desired quantity of loose objects 11 is present in the metering device 15, the command and control unit 28 drives the interception valve 19 which opens the unloading side 17 of the metering device 15 releasing the loose objects 11 into the blowing sub-circuit 21, in particular into the sending pipe 53, through the outlet member 25.

In this way, the loose objects 11 are automatically transferred by blowing of the pneumatic device 26 toward the loading zone 13 of the treatment chamber 12.

In this step, that is, in the sending condition, for example, the valves 27 will close the first suction intake 22 and open the second suction intake 23 toward the external environment so as to prevent other loose objects 11 from reaching the metering device 15 as a result of the suction, that is, of the depression created by the first suction intake 22 in the metering device 15 starting from the source 33 of loose objects 11.

Furthermore, in this step, the blocking valve 32 will possibly be closed, thus closing the feed pipe 18, so as to further prevent the loose objects 11 from entering into the metering device 15 during the step of blowing the loose objects 11 metered to the loading zone 13.

Subsequently, the loose objects 11 will be released by gravity inside the treatment chamber 12 through the loading zone 13 by means of the introduction assembly 51. Of course, in this step, the stop valve 42 positioned in the loading zone 13 will be kept open and the quantity of loose objects 11 will be introduced.

For example, once the loading of loose objects 11 into one containing sector is completed, the drum rotates around the shaft 37 so as to present another containing sector in correspondence with the loading zone 13.

Once the loading of one containing sector is finished, the control device 43 can automatically send a signal of "load completed" to the command and control unit 28 of the treatment machine 14, so that, for example, it again activates a functioning cycle of the feed apparatus 10 to fill a new containing sector.

A new transfer then follows and, therefore, a step of sucking in loose objects 11 from the source 33 of loose objects 11 along the feed pipe 18 to the metering device 15 in the removal condition, so as to be able to send another quantity of loose objects 11 suitably metered to the other containing sector in correspondence with the loading zone 13.

In this step of sucking in the loose objects 11 from the source 33 of loose objects 11 to the metering device 15, the valves 27 will close the second suction intake 23 and open the first suction intake 22 so as to suck in loose objects 11 from the source 33 of loose objects 11 to the metering device 15 by means of the feed pipe 18.

In this step, moreover, the interception valve 19 associated with the unloading side 17 of the metering device 15 is closed, so as to prevent the fall of the loose objects 11 not yet correctly metered into the sending pipe 53.

Furthermore, in this step, the blocking valve 32 will be opened, thus opening the feed pipe 18, so as to allow the transfer of the loose objects 11 toward the metering device 15.

It is also clear that, although the present invention has been described with reference to some specific examples, a person of skill in the art shall certainly be able to achieve many other equivalent forms of apparatus 10 to feed loose objects to a treatment machine and corresponding feed method, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Feed apparatus to feed loose objects (11) to a treatment machine (14) able to treat loose objects (11) and comprising a treatment chamber (12) provided with a loading zone (13) of said loose objects (11), said feed apparatus comprising:
- a source (33) of loose objects (11);
- a metering device (15) which has a loading side (16) connected to said source (33) of loose objects (11) by means of a feed pipe (18) of said loose objects (11) and an unloading side (17) with which an interception valve (19) is associated, said metering device (15) being configured to accommodate a determinate quantity of loose objects (11);
- an introduction assembly (51) connected to said metering device (15) by means of a sending pipe (53) connected to an outlet member (25) present on the unloading side (17) of said metering device (15), said introduction assembly (51) being configured to introduce the loose objects (11) into said loading zone (13), directly in the treatment chamber (12),
- a pneumatic feed circuit (50) selectively drivable alternately in a removal condition and a condition for sending said loose objects (11) respectively from the source (33) to the metering device (15), through the feed pipe (18), and from the metering device (15) to the introduction assembly (51),
- a pneumatic device (26) connected to said pneumatic feed circuit (50) and configured to suck in loose objects (11) from the source (33) to the metering device (15) in said removal condition and to blow loose objects (11) from the metering device (15) to the introduction assembly (51), **characterized in that** that said pneumatic feed circuit (50) comprises:
- a suction sub-circuit (20) which includes said feed pipe (18), a first suction pipe (54) connected to the loading side (16) of the metering device (15), a second suction pipe (56) connected to an external environment and a main suction pipe (55) connected on one side to said first suction pipe (54) and to said second suction pipe (56) and on another side connected to a suction door (57) of said pneumatic device (26), respective valves (27) being mounted on said first suction pipe (54) and on said second suction pipe (56), said suction sub-circuit (20) being provided with a first suction intake (22), provided in said source (33), associated with said pneumatic device (26) and connected to said feed pipe (18), and with a second suction intake (23), provided terminally on said second suction pipe (56), associated with said pneumatic device (26) and connected to said external environment, said valves (27) being configured to alternately close said second suction pipe (56) and said first suction pipe (54) respectively in said removal condition and in said condition for sending said loose objects (11).

2. Feed apparatus as in claim 1, **characterized in that** said pneumatic feed circuit (50) comprises:
- a blowing sub-circuit (21) which includes said sending pipe (53), a delivery intake (24) present on a blowing door (58) of said pneumatic device (26), a blowing pipe (59) connected to said sending pipe (53) and to said outlet member (25) present on the unloading side (17) of said metering device (15), an interception valve (19) mounted on said outlet member (25) which is configured to close said outlet member (25) in said removal condition and to open said outlet member (25) in said sending condition.

3. Feed apparatus as in claims 1 and 2, **characterized in that** said interception valve (19) is selectively openable/closable in synchrony with the respective opening/closing of the valve (27b) mounted on said second suction pipe (56) and the closing/opening of the valve (27a) mounted on said first suction pipe (54).

4. Feed apparatus as in any claim hereinbefore, **characterized in that** it comprises a command and control unit (28) configured to control said pneumatic feed circuit (50) in correlation with said pneumatic device (26) to selectively determine at least said removal condition and said sending condition.

5. Feed apparatus as in claim 4, **characterized in that** said command and control unit (28) is configured to control the opening/closing synchrony of said interception valve (19) with respect to the valve (27b) mounted on said second suction pipe (56) and to the valve (27a) mounted on said first suction pipe (54).

6. Feed apparatus as in any claim hereinbefore, **characterized in that** said source (33) of loose objects (11) and said metering device (15) are installed in a common feed area (52) opposite said treatment machine (14).

7. Feed apparatus as in any claim hereinbefore, **characterized in that** said introduction assembly (51) comprises a telescopic connection pipe (44) transverse to the treatment chamber (12) and drivable to be selectively lengthened and retracted with respect to the treatment chamber (12) in order to be positioned, with a terminal portion thereof, inside the treatment chamber (12) itself.

8. Feed apparatus as in any claim hereinbefore, **characterized in that** said introduction assembly (51) comprises a deflector element (45) disposed before an exit portion (48) of the sending pipe (53) configured to vent the blowing of the pneumatic device (26), said deflector element (45) deflecting the loose objects (11) in the sending condition, toward the loading zone (13) before reaching the exit portion (48).

9. Treatment plant comprising a treatment machine (14) able to treat loose objects (11) and a feed apparatus (10) for loose objects (11), as defined in any claim hereinbefore, to feed said loose objects (11) to be treated to said treatment machine (14).

10. Treatment plant as in claim 9, **characterized in that** said source (33) of loose objects (11) and said metering device (15) are installed in a common feed area (52) opposite said treatment machine (14).

11. Feed method to feed loose objects (11) to a treatment machine (14) able to treat loose objects (11) in a treatment chamber (12) provided with a loading zone (13) of said loose objects (11), **characterized in that** said method comprises:
- suction of a metered quantity of loose objects (11) from a source (33) of loose objects (11) in a metering device (15) by means of a pneumatic feed circuit (50) driven in a condition of removing said loose objects (11) from the source (33) to the metering device (15) using a pneumatic device (26) connected to said pneumatic feed circuit (50) to suck in loose objects (11) from the source (33) to the metering device (15);
- blowing the metered quantity of loose objects (11) present in the metering device (15) from the metering device (15) by means of said pneumatic feed circuit (50) driven in a condition for sending said loose objects (11) from the metering device (15) to an introduction assembly (51) which introduces the loose objects (11), in said loading zone (13), directly into the treatment chamber (12), using said pneumatic device (26) to blow the loose objects (11) from the metering device (15) to the introduction assembly (51), wherein said pneumatic feed circuit (50) comprises: a suction sub-circuit (20) which includes said feed pipe (18), a first suction pipe (54) connected to the loading side (16) of the metering device (15), a second suction pipe (56) connected to an external environment and a main suction pipe (55) connected on one side to said first suction pipe (54) and to said second suction pipe (56), and on another side connected to a suction door (57) of said pneumatic device (26), respective valves (27) being mounted on said first suction pipe (54) and on said second suction pipe (56), said suction sub-circuit (20) being provided with a first suction intake (22), provided in said source (33), associated with said pneumatic device (26) and connected to said feed pipe (18) and with a second suction intake (23), provided terminally on said second suction pipe (56), associated with said pneumatic device (26) and connected to said external environment, said valves (27) being configured to alternately close said second suction pipe (56) and said first suction pipe (54) respectively in said removal condition and in said condition for sending said loose objects (11).

## Patentansprüche

1. Zuführvorrichtung zum Zuführen von losen Gegenständen (11) zu einer Bearbeitungsmaschine (14), die zur Bearbeitung von losen Gegenständen (11) imstande ist und die eine Bearbeitungskammer (12) aufweist, die mit einem Beladebereich (13) für die losen Gegenstände (11) versehen ist, wobei die Zuführvorrichtung aufweist:
- eine Quelle (33) für lose Gegenstände (11);
- eine Dosiervorrichtung (15), die eine Beladeseite (16), die über eine Zuführleitung (18) für lose Gegenstände (11) mit der Quelle (33) für lose Gegenstände (11) verbunden ist, und eine Entladeseite (17) hat, mit welcher ein Absperrventil (19) verknüpft ist, wobei die Dosiervorrichtung (15) konfiguriert ist, um eine bestimmte Menge an losen Gegenständen (11) aufzunehmen;
- eine Einführbaugruppe (51), die mit der Dosiervorrichtung (15) verbunden ist über eine Förderleitung (53), die mit einem an der Entladeseite (17) der Dosiervorrichtung (15) vorhandenen Auslasselement (25) verbunden ist, wobei die Einführbaugruppe (51) konfiguriert ist, um die losen Gegenstände (11) in den Beladebereich (13) direkt in der Bearbeitungskammer (12) einzuführen,
- einen pneumatischen Zuführkreis (50), der selektiv abwechselnd betreibbar ist in einem Entnahmezustand und in einem Zustand zum Fördern der losen Gegenstände (11) zugeordnet von der Quelle (33) zur Dosiervorrichtung (15) durch die Zuführleitung (18) sowie von der Dosiervorrichtung (15) zur Einführbaugruppe (51),
- eine pneumatische Vorrichtung (26), die mit dem pneumatischen Zuführkreis (50) verbunden ist und konfiguriert ist, um in dem Entnahmezustand lose Gegenstände (11) von der Quelle (33) zur Dosiervorrichtung (15) anzusaugen und um lose Gegenstände (11) von der Dosiervorrichtung (15) zur Einführbaugruppe (51) zu blasen, **dadurch gekennzeichnet, dass** der pneumatische Zuführkreis (50) aufweist:
- einen Ansaugsubkreis (20), der die Zuführleitung (18), eine erste Ansaugleitung (54), die mit der Beladeseite (16) der Dosiervorrichtung (15) verbunden ist, eine zweite Ansaugleitung (56), das mit der Außenumgebung verbunden ist, und eine Hauptansaugleitung (55) aufweist, die auf einer Seite mit der ersten Ansaugleitung (54) und mit der zweiten Ansaugleitung (56) verbunden ist und die auf der anderen Seite mit einer Saugklappe (57) der pneumatischen Vorrichtung (26) verbunden ist, wobei jeweilige Ventile (27) an der ersten Ansaugleitung (54) und an der zweiten Ansaugleitung (56) angebracht sind, wobei der Ansaugsubkreis (20) versehen ist mit einem ersten Ansaugeinlass (22), der in der Quelle (33) vorgesehen ist, mit der pneumatischen Vorrichtung (26) verknüpft ist und mit der Zuführleitung (18) verbunden ist, sowie mit einem zweiten Ansaugeinlass (23), der endseitig an der zweiten Ansaugleitung (56) vorgesehen ist , mit der pneumatischen Vorrichtung (26) verknüpft ist und mit der Außenumgebung verbunden ist, wobei die Ventile (27) konfiguriert sind, um abwechselnd die zweite Ansaugleitung (56) und die erste Ansaugleitung (54) zugeordnet im Entnahmezustand und im Zustand zum Fördern der losen Gegenstände (11) zu verschließen.

2. Zuführvorrichtung nach Anspruch 1, **dadurch gekennzeichnet, dass** der pneumatische Zuführkreis (50) aufweist:
- einen Blassubkreis (21), der die Förderleitung (53), einen an einer Blasklappe (58) der pneumatischen Vorrichtung (26) vorhandenen Zufuhreinlass (24), eine mit der Förderleitung (53) und dem an der Entladeseite (17) der Dosiervorrichtung (15) vorhandenen Auslasselement (25) verbundene Blasleitung (59) und ein an dem Auslasselement (25) angebrachtes Absperrventil (19) aufweist, das konfiguriert ist, um das Auslasselement (25) im Entnahmezustand zu verschließen und das Auslasselement (25) im Zuführen-Zustand zu öffnen.

3. Zuführvorrichtung nach den Ansprüchen 1 und 2, **dadurch gekennzeichnet, dass** das Absperrventil (19) selektiv geöffnet/geschlossen werden kann synchron mit dem jeweiligen Öffnen/Schließen des an der zweiten Ansaugleitung (56) angebrachten Ventils (27b) und dem Schließen/Öffnen des an der ersten Ansaugleitung (54) angebrachten Ventils (27a).

4. Zuführvorrichtung nach irgendeinem vorstehenden Anspruch, **dadurch gekennzeichnet, dass** sie eine Befehls- und Steuereinheit (28) aufweist, die konfiguriert ist, um den pneumatischen Zuführkreis (50) in Korrelation mit der pneumatischen Vorrichtung (26) zu steuern, um zumindest den Entnahmezustand und den Zuführen-Zustand selektiv festzulegen.

5. Zuführvorrichtung nach Anspruch 4**, dadurch gekennzeichnet, dass** die Befehls- und Steuereinheit (28) konfiguriert ist, um die Öffnen/Schließen-Synchronität des Absperrventils (19) in Bezug auf das an der zweiten Ansaugleitung (56) angebrachte Ventil (27b) und auf das an der ersten Ansaugleitung (54) angebrachte Ventil (27a) zu steuern.

6. Zuführvorrichtung nach irgendeinem vorstehenden Anspruch, **dadurch gekennzeichnet, dass** die Quelle (33) für lose Gegenstände (11) und die Dosiervorrichtung (15) in einem gemeinsamen Zuführbereich (52) entgegengesetzt zu der Bearbeitungsmaschine (14) installiert sind.

7. Zuführvorrichtung nach irgendeinem vorstehenden Anspruch, **dadurch gekennzeichnet, dass** die Einführbaugruppe (51) eine teleskopische Verbindungsleitung (44) aufweist, die quer zur Bearbeitungskammer (12) verläuft und die so betreibbar ist, dass sie in Bezug auf die Bearbeitungskammer (12) selektiv verlängert und eingefahren werden kann, um mit einem Endabschnitt davon innerhalb der Bearbeitungskammer (12) ihrerseits positioniert zu werden

8. Zuführvorrichtung nach irgendeinem vorstehenden Anspruch, **dadurch gekennzeichnet, dass** die Einführbaugruppe (51) ein Ablenkelement (45) aufweist, das vor einem Austrittsabschnitt (48) der Förderleitung (53) angeordnet ist, konfiguriert, um den Luftstrom der pneumatischen Vorrichtung (26) zu entlüften, wobei das Ablenkelement (45) die losen Gegenstände (11) im Fördern-Zustand in Richtung zum Beladebereich (13) umlenkt, bevor sie den Austrittsabschnitt (48) erreichen.

9. Bearbeitungsanlage, aufweisend eine Bearbeitungsmaschine (14), die imstande ist, lose Gegenstände (11) zu bearbeiten, und eine Zuführvorrichtung (10) für lose Gegenstände (11) wie in irgendeinem vorstehenden Anspruch definiert, um die zu bearbeitenden losen Gegenstände (11) der Bearbeitungsmaschine (14) zuzuführen.

10. Bearbeitungsanlage nach Anspruch 9, **dadurch gekennzeichnet, dass** die Quelle (33) für lose Gegenstände (11) und die Dosiervorrichtung (15) in einem gemeinsamen Zuführbereich (52) entgegengesetzt zu der Bearbeitungsmaschine (14) installiert sind.

11. Zuführverfahren zum Zuführen von losen Gegenständen (11) zu einer Bearbeitungsmaschine (14), die imstande ist, lose Gegenstände (11) in einer Bearbeitungskammer (12) zu bearbeiten, die mit einem Beladebereich (13) für die losen Gegenstände (11) versehen ist, **dadurch gekennzeichnet, dass** das Verfahren aufweist:
- Ansaugen einer dosierten Menge an losen Gegenständen (11) von einer Quelle (33) für lose Gegenstände (11) in eine Dosiervorrichtung (15) mittels eines pneumatischen Zuführkreises (50), der betrieben wird in einem Zustand des Entnehmens der losen Gegenstände (11) von der Quelle (33) zur Dosiervorrichtung (15) unter Verwendung einer pneumatischen Vorrichtung (26), die mit dem pneumatischen Zuführkreis (50) verbunden ist, um lose Gegenstände (11) von der Quelle (33) aus in die Dosiervorrichtung (15) anzusaugen;
- Blasen der in der Dosiervorrichtung (15) befindlichen dosierten Menge an losen Gegenständen (11) von der Dosiervorrichtung (15) aus mittels des pneumatischen Zuführkreises (50), der betrieben wird in einem Zustand zum Fördern der losen Gegenstände (11) von der Dosiervorrichtung (15) aus zu einer Einführbaugruppe (51), die die losen Gegenstände (11) in der Beladezone (13) direkt in der Bearbeitungskammer (12) einführt, unter Verwendung der pneumatischen Vorrichtung (26), um die losen Gegenstände (11) von der Dosiervorrichtung (15) aus zur Einführbaugruppe (51) zu blasen, wobei der pneumatische Zuführkreis (50) aufweist: einen Ansaugsubkreis (20), der die Förderleitung (18) aufweist, eine erste Ansaugleitung (54), die mit der Beladeseite (16) der Dosiervorrichtung (15) verbunden ist, eine zweite Ansaugleitung (56), die mit einer Außenumgebung verbunden ist, und eine Hauptansaugleitung (55), die auf einer Seite mit der ersten Ansaugleitung (54) und der zweiten Ansaugleitung (56) verbunden ist, und auf der anderen Seite mit einer Ansaugklappe (57) der pneumatischen Vorrichtung (26) verbunden ist, wobei an der ersten Ansaugleitung (54) und an der zweiten Ansaugleitung (56) jeweilige Ventile (27) angebracht sind, wobei der Ansaugsubkreis (20) versehen ist mit einem ersten Ansaugeinlass (22), der in der Quelle (33) vorgesehen ist, mit der pneumatischen Vorrichtung (26) verknüpft ist und mit der Zuführleitung (18) verbunden ist, und mit einem zweiten Ansaugeinlass (23), der endseitig an der zweiten Ansaugleitung (56) vorgesehen ist, mit der pneumatischen Vorrichtung (26) verknüpft ist und mit der Außenumgebung verbunden ist, wobei die Ventile (27) konfiguriert sind, um abwechselnd die zweite Ansaugleitung (56) und die erste Ansaugleitung (54) zugeordnet im Entnahmezustand und im Zustand zum Fördern der losen Gegenstände (11) zu verschließen.

## Revendications

1. Appareil d'alimentation pour alimenter en objets en vrac (11) une machine de traitement (14) apte à traiter des objets en vrac (11) et comprenant une chambre de traitement (12) munie d'une zone de chargement (13) desdits objets en vrac (11), ledit appareil d'alimentation comprenant :
- une source (33) d'objets en vrac (11) ;
- un dispositif de mesure (15) qui présente un côté chargement (16) relié à ladite source (33) d'objets en vrac (11) au moyen d'un tuyau d'alimentation (18) en objets en vrac (11) et un côté déchargement (17) auquel une vanne d'interception (19) est associée, ledit dispositif de mesure (15) étant configuré pour recevoir une quantité déterminée d'objets en vrac (11) ;
- un ensemble d'introduction (51) relié audit dispositif de mesure (15) au moyen d'un tuyau d'envoi (53) relié à un organe de sortie (25) présent sur le côté déchargement (17) dudit dispositif de mesure (15), ledit ensemble d'introduction (51) étant configuré pour introduire les objets en vrac (11) dans ladite zone de chargement (13), directement dans la chambre de traitement (12),
- un circuit d'alimentation pneumatique (50) actionnable sélectivement de manière alternée dans une condition de prélèvement et dans une condition pour envoyer lesdits objets en vrac (11) respectivement de la source (33) au dispositif de mesure (15), à travers le tuyau d'alimentation (18), et du dispositif de mesure (15) à l'ensemble d'introduction (51),
- un dispositif pneumatique (26) relié audit circuit d'alimentation pneumatique (50) et configuré pour aspirer des objets en vrac (11) de la source (33) au dispositif de mesure (15) dans ladite condition de prélèvement et pour souffler des objets en vrac (11) du dispositif de mesure (15) à l'ensemble d'introduction (51), **caractérisé en ce que** ledit circuit d'alimentation pneumatique (50) comprend :
- un sous-circuit d'aspiration (20) qui inclut ledit tuyau d'alimentation (18), un premier tuyau d'aspiration (54) relié au côté chargement (16) du dispositif de mesure (15), un second tuyau d'aspiration (56) relié à un environnement extérieur et un tuyau d'aspiration principal (55) relié d'un côté audit premier tuyau d'aspiration (54) et audit second tuyau d'aspiration (56) et relié de l'autre côté à une porte d'aspiration (57) dudit dispositif pneumatique (26), des vannes respectives (27) étant montées sur ledit premier tuyau d'aspiration (54) et sur ledit second tuyau d'aspiration (56), ledit sous-circuit d'aspiration (20) étant muni d'une première entrée d'aspiration (22), disposée dans ladite source (33), associée audit dispositif pneumatique (26) et reliée audit tuyau d'alimentation (18), et d'une seconde entrée d'aspiration (23), disposée en fin dudit second tuyau d'aspiration (56), associée audit dispositif pneumatique (26) et reliée audit environnement extérieur, lesdites vannes (27) étant configurées pour fermer alternativement ledit second tuyau d'aspiration (56) et ledit premier tuyau d'aspiration (54) respectivement dans ladite condition de prélèvement et dans ladite condition pour envoyer lesdits objets en vrac (11).

2. Appareil d'alimentation selon la revendication 1, **caractérisé en ce que** ledit circuit d'alimentation pneumatique (50) comprend :
- un sous-circuit de soufflage (21) qui inclut ledit tuyau d'envoi (53), une entrée de distribution (24) présente sur une porte de soufflage (58) dudit dispositif pneumatique (26), un tuyau de soufflage (59) relié audit tuyau d'envoi (53) et audit organe de sortie (25) présent sur le côté déchargement (17) dudit dispositif de mesure (15), une vanne d'interception (19) montée sur ledit organe de sortie (25) qui est configurée pour fermer ledit organe de sortie (25) dans ladite condition de prélèvement et pour ouvrir ledit organe de sortie (25) dans ladite condition d'envoi.

3. Appareil d'alimentation selon les revendications 1 et 2, **caractérisé en ce que** ladite vanne d'interception (19) peut être sélectivement ouverte/fermée en synchronisation avec l'ouverture/fermeture respective de la vanne (27b) montée sur ledit second tuyau d'aspiration (56) et la fermeture/ouverture de la vanne (27a) montée sur ledit premier tuyau d'aspiration (54).

4. Appareil d'alimentation selon une quelconque revendication précédente, **caractérisé en ce qu'**il comprend une unité d'instruction et de commande (28) configurée pour commander ledit circuit d'alimentation pneumatique (50) en corrélation avec ledit dispositif pneumatique (26) pour déterminer sélectivement au moins ladite condition de prélèvement et ladite condition d'envoi.

5. Appareil d'alimentation selon la revendication 4, **caractérisé en ce que** ladite unité d'instruction et de commande (28) est configurée pour commander la synchronisation d'ouverture/fermeture de ladite vanne d'interception (19) par rapport à la vanne (27b) montée sur ledit second tuyau d'aspiration (56) et à la vanne (27a) montée sur ledit premier tuyau d'aspiration (54).

6. Appareil d'alimentation selon une quelconque revendication précédente, **caractérisé en ce que** ladite source (33) d'objets en vrac (11) et ledit dispositif de mesure (15) sont installés dans une zone d'alimentation commune (52) opposée à ladite machine de traitement (14).

7. Appareil d'alimentation selon une quelconque revendication précédente, **caractérisé en ce que** ledit ensemble d'introduction (51) comprend un tuyau de liaison télescopique (44) transversal à la chambre de traitement (12) et actionnable pour être sélectivement allongé et rétracté par rapport à la chambre de traitement (12) afin d'être positionné, avec une partie terminale de celui-ci, à l'intérieur de la chambre de traitement (12) elle-même.

8. Appareil d'alimentation selon une quelconque revendication précédente, **caractérisé en ce que** ledit ensemble d'introduction (51) comprend un élément déflecteur (45) disposé avant une partie de sortie (48) du tuyau d'envoi (53) configuré pour ventiler le soufflage du dispositif pneumatique (26), ledit élément déflecteur (45) déviant les objets en vrac (11) dans la condition d'envoi, vers la zone de chargement (13) avant qu'ils n'atteignent la partie de sortie (48).

9. Installation de traitement comprenant une machine de traitement (14) apte à traiter des objets en vrac (11) et un appareil d'alimentation (10) pour des objets en vrac (11), selon une quelconque revendication précédente, pour alimenter en objets en vrac (11) à traiter ladite machine de traitement (14).

10. Installation de traitement selon la revendication 9, **caractérisée en ce que** ladite source (33) d'objets en vrac (11) et ledit dispositif de mesure (15) sont installés dans une zone d'alimentation commune (52) opposée à ladite machine de traitement (14).

11. Procédé d'alimentation pour alimenter en objets en vrac (11) une machine de traitement (14) apte à traiter des objets en vrac (11) dans une chambre de traitement (12) munie d'une zone de chargement (13) desdits objets en vrac (11), **caractérisé en ce que** ledit procédé comprend :
- l'aspiration d'une quantité mesurée d'objets en vrac (11) à partir d'une source (33) d'objets en vrac (11) dans un dispositif de mesure (15) au moyen d'un circuit d'alimentation pneumatique (50) actionné dans une condition de prélèvement desdits objets en vrac (11) de la source (33) au dispositif de mesure (15) à l'aide d'un dispositif pneumatique (26) relié audit circuit d'alimentation pneumatique (50) pour aspirer des objets en vrac (11) de la source (33) au dispositif de mesure (15) ;
- le soufflage de la quantité mesurée d'objets en vrac (11) présents dans le dispositif de mesure (15) depuis le dispositif de mesure (15) au moyen dudit circuit d'alimentation pneumatique (50) actionné dans une condition pour envoyer lesdits objets en vrac (11) du dispositif de mesure (15) à un ensemble d'introduction (51) qui introduit les objets en vrac (11), dans ladite zone de chargement (13), directement dans la chambre de traitement (12), en utilisant ledit dispositif pneumatique (26) pour souffler les objets en vrac (11) du dispositif de mesure (15) à l'ensemble d'introduction (51), dans lequel ledit circuit d'alimentation pneumatique (50) comprend : un sous-circuit d'aspiration (20) qui inclut ledit tuyau d'alimentation (18), un premier tuyau d'aspiration (54) relié au côté chargement (16) du dispositif de mesure (15), un second tuyau d'aspiration (56) relié à un environnement extérieur et un tuyau d'aspiration principal (55) relié d'un côté audit premier tuyau d'aspiration (54) et audit second tuyau d'aspiration (56), et relié de l'autre côté à une porte d'aspiration (57) dudit dispositif pneumatique (26), des vannes respectives (27) étant montées sur ledit premier tuyau d'aspiration (54) et sur ledit second tuyau d'aspiration (56), ledit sous-circuit d'aspiration (20) étant muni d'une première entrée d'aspiration (22), disposée dans ladite source (33), associée audit dispositif pneumatique (26) et reliée audit tuyau d'alimentation (18) et d'une seconde entrée d'aspiration (23), disposée en fin sur ledit second tuyau d'aspiration (56), associée audit dispositif pneumatique (26) et reliée audit environnement extérieur, lesdites vannes (27) étant configurées pour fermer alternativement ledit second tuyau d'aspiration (56) et ledit premier tuyau d'aspiration (54) respectivement dans ladite condition de prélèvement et dans ladite condition pour envoyer lesdits objets en vrac (11).
